(19) Europäisches Patentamt / European Patent Office / Office européen des brevets

(11) **EP 3 110 822 B1**

(12) **EUROPÄISCHE PATENTSCHRIFT**

(45) Veröffentlichungstag und Bekanntmachung des Hinweises auf die Patenterteilung:
**07.03.2018 Patentblatt 2018/10**

(21) Anmeldenummer: **16713892.4**

(22) Anmeldetag: **01.04.2016**

(51) Int Cl.:
*C07F 7/18* *(2006.01)*    *C08G 77/00* *(2006.01)*
*C03C 25/00* *(2018.01)*

(86) Internationale Anmeldenummer:
**PCT/EP2016/057183**

(87) Internationale Veröffentlichungsnummer:
**WO 2016/188654 (01.12.2016 Gazette 2016/48)**

(54) **VERFAHREN ZUR HERSTELLUNG VON WÄSSRIGEN HYDROLYSATEN VON AMINOALKYLTRIALKOXYSILANEN**

METHOD FOR THE PREPARATION OF AQUEOUS HYDROLYSATES OF AMINOALKYLTRI ALKOXYSILANES

PROCÉDÉ DE FABRICATION D'HYDROLYSATS AQUEUX À PARTIR D'AMINOALKYLTRIALCOXYSILANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.05.2015 EP 15169684**

(43) Veröffentlichungstag der Anmeldung:
**04.01.2017 Patentblatt 2017/01**

(73) Patentinhaber: **Evonik Degussa GmbH**
**45128 Essen (DE)**

(72) Erfinder:
• **STANDKE, Burkhard**
**79540 Lörrach (DE)**
• **WASSMER, Christian**
**79688 Hausen (DE)**
• **LIPPERT, Irene**
**79618 Rheinfelden (DE)**
• **BIBBO, Kerstin**
**79618 Rheinfelden (DE)**

(56) Entgegenhaltungen:
**EP-A2- 1 031 593    US-B2- 6 512 132**

**Beschreibung**

Die vorliegende Erfindung betrifft ein Verfahren zur Herstellung von wässrigen Hydrolysaten von Aminoalkyltrialkoxysilanen, das zu konzentrierten wässrigen Lösungen dieser Aminoalkyltrialkoxysilane führt, die im Wesentlichen frei von Alkoholen sind, und in besonders wirtschaftlicher Weise durchführbar ist.

Aus dem Stand der Technik sind Verfahren bekannt, bei denen Aminoalkyltrialkoxysilane, wie zum Beispiel 3-Aminopropyltriethoxisilane, mit zusätzlichen hydrolysierbaren Silanen, wie zum Beispiel Bis-triethoxysilylpropylamin und/oder Alkyltrialkoxysilane und/oder funktionell substituierte Alkyltrialkoxysilane, wobei sich die funktionelle Gruppe von der Aminogruppe unterscheidet, cohydrolysiert werden. Solche Verfahren sind zum Beispiel aus EP-A 675 128, EP-A 716 127, EP-A 997 469, EP-A 1 031 593 und WO 2009/03538 bekannt. Im Gegensatz hierzu betrifft die vorliegende Erfindung ein Verfahren zur Herstellung von Hydrolysaten von Aminoalkyltrialkoxysilanen mit einem höheren Feststoffgehalt und bei denen keine weiteren hydrolysierbaren Silane, die sich von Aminoalkyltrialkoxysilanen unterscheiden, zugegen sind.

Die DE 103 33 941 A1 betrifft eine Schlichtenzusammensetzung für Glasfasern, die neben Wasser einen Filmbildner und einen Haftvermittler enthalten. Als Haftvermittler wird ein Hydrolysat von 3-Aminopropyltriethoxysilan vorgeschlagen. In dem einzigen Ausführungsbeispiel wird ohne nähere Angaben zur Verfahrensführung γ-Aminopropyltriethoxysilan mit einem mehr als 400fachen molaren Überschuss an Wasser hydrolysiert.

EP-A 1 017 880 offenbart ein Verfahren zur Beschichtung eines Metallsubstrats, bei dem eine Behandlungslösung auf die Oberfläche des Metallsubstrats aufgebracht wird, wobei die Behandlungslösung ein teilweise hydrolysiertes Aminosilan und eine fluorhaltige anorganische Säure in einem Verhältnis von 1:2 bis 2:1 enthält. Das partiell hydrolysierte Aminosilan wird durch Zugeben sehr geringer Mengen von Wasser im Bereich von 4 bis 5 %, bezogen auf das Gesamtvolumen von Wasser und Aminosilan, durch Stehenlassen über Nacht hydrolysiert.

US-A 3,810,843 betrifft die Herstellung von Zusammensetzungen durch In-Kontakt-Bringen eines Kieselsäuresols mit einem organofunktionellen Silankupplungsmittel, das vorher in einem wässrigen System hydrolisiert worden ist. Unter anderem wird 3-Aminopropyltriethoxysilan als Kupplungsmittel vorgeschlagen. Die allgemeine Beschreibung der Druckschrift enthält keine Angaben über das Verfahren, mit dem das Hydrolysat des Silankupplungsmittels hergestellt wird. In einer konkreten Ausführungsform wird 3-Aminopropyltriethoxysilan einer verdünnten wässrigen Natronlauge zugegeben, wobei das molare Verhältnis von Wasser zu 3-Aminopropyltriethoxysilan 26,7 % beträgt.

Zengping Zhang et al. beschreibt in "Journal of Applied Polymer Science", 103. Auflage, 2608-2614 (2007), die Herstellung von Octa(aminopropylsilsequioxanen) mit Käfigstruktur. Hierbei wird zum Beispiel 3-Aminopropyltriethoxysilan in einer Mischung von Acetonitril und 1-Propanol in Gegenwart von Tetraethylammoniumhydroxid hydrolisiert, wobei das molare Verhältnis von Wasser zu Silan 8,9 : 1 beträgt, um die gewünschten Käfigstrukturen herzustellen.

Die US-A 6,512,132 betrifft ebenfalls ein Verfahren zur Herstellung wässriger Hydrolysate von 3-Aminopropyltriethoxysilan. Zielsetzung ist ebenfalls die Bereitstellung solcher Hydrolysate, die im Wesentlichen frei von organischen Lösungsmitteln, insbesondere Alkohol, ist und eine hohe Lagerstabilität aufweisen. Die Lagerstabilität wird durch Messung der Farbzahl nach Lagerung über einen definierten Zeitraum bestimmt. Gemäß der allgemeinen Lehre der Druckschrift wird die wässrige Lösung durch Umsetzen 1 mol Aminoalkylsilans mit 1,5 - 10 mol Wasser und Abdestillieren der flüchtigen organischen Verbindung, die durch die Hydrolysereaktion gebildet wird, hergestellt. In dem konkreten Ausführungsbeispiel wird das 3-Aminopropyltriethoxysilan vorgelegt und mit Wasser versetzt. Gemäß der allgemeinen Lehre wird die Hydrolyse in einem Temperaturbereich von 0 °C bis 150 °C durchgeführt. Die konkreten Ausführungsformen enthalten keine Angabe über die Hydrolysierungstemperatur. Im Gegensatz zur allgemeinen Lehre wird in Beispiel 2 das 3-Aminopropyltriethoxysilan mit Wasser in einem molaren Verhältnis von Wasser zu Silan von 12,4 durchgeführt. Gemäß der Lehre von US 6,512,132 werden zwar wässrige Hydrolysate von 3-Aminoalkyltriethoxysilan in hohen Konzentrationen in der Größenordnung von 50 Gew.-% erhalten, die auch weitgehend frei von durch Hydrolyse entstandenem Alkohol sind. Allerdings führt das beschriebene Verfahren zu einer vergleichsweise niedriger Raum/Zeit-Ausbeute. So ist bei der Verfahrensweise gemäß US 6,512,132 von großem Nachteil, dass eine solche Herstellungsweise besagter Produkte eine lange Dosier- bzw. Rührzeit der erforderlichen Wassermenge bedingt und darüber hinaus während des Herstellprozesses regelmäßig starke Ausflockungen bzw. Ausscheidungen verbunden mit einem erheblichen Viskositätsanstieg auftreten, was einen längeren und energieintensiveren Rühraufwand zur Folge hat, um eine hinreichende Produkthomogenität zu erzielen. Darüber hinaus können solche Ausscheidungen in der Praxis den Produktionsbetrieb durch Ablagerungen oder Verstopfungen, verbunden mit notwendigen Stillstandzeiten für Reinigungsmaßnahmen, maßgebend stören.

Im Handel sind ebenfalls wässrige Hydrolysate von 3-Aminopropylsilanen erhältlich, wie zum Beispiel Dynasylan® HYDROSIL 1151. Diese wässrigen Lösungen sind ebenfalls weitgehend frei von organischen Lösungsmitteln. Der Feststoffgehalt dieser Lösungen ist aber eher gering und liegt im Bereich von 20 Gew.-%. Mit dieser niedrigen Konzentration an hydrolysiertem Silan der kommerziellen Produkte sind verschiedene Nachteile verbunden. Dies sind insbesondere erhöhte Transportkosten, da viel Wasser und wenig Wirkstoff transportiert wird. Weiterhin gefrieren die verdünnten Lösungen bei geringen Minusgraden, so dass bei niedrigen Außentemperaturen hohe Transportkosten durch den notwendigen Wärmetransport entstehen. Die verdünnten Produkte enthalten viel Wasser und sind damit in Anwendung, bei denen hohe Mengen an Wasser nachteilig sind, nicht einsetzbar. Weiterhin lassen sich mit verdünnten Lösungen keine Konzentrate formulieren.

Aufgabe der vorliegenden Erfindung ist es daher, ein spezielles, möglichst wirtschaftliches Verfahren bereitzustellen, das unter vergleichbaren apparativen Bedingungen bei zumindest vergleichsweise hohen oder besseren Raum/Zeit-Ausbeuten zu höher konzentrierten Hydrolysaten von Aminoalkyltrialkoxysilanen, d.h. Feststoffgehalt, führt und eine so erhaltene Zusammensetzung gleichzeitig im Wesentlichen frei von organischen Lösungsmitteln ist, d.h. insbesondere einen möglichst geringen VOC (Volatile Organic Compounds = flüchtige organische Verbindungen) aufweist.

Nachfolgend werden besagte Aminoalkyltrialkoxysilane auch hydrolysierbare Silane oder kurz Silane genannt.

Diese Aufgabe wurde in überraschender Weise durch ein spezielles Verfahren zur Herstellung von wässrigen Hydrolysaten von Aminoalkyltrialkoxysilanen durch

(a) Vorlegen von Wasser, optional Erwärmen,
(b) Zugeben von hydrolisierbaren Silanen bestehend aus mindestens einem Aminoalkyltrialkoxysilan in einer Menge, die ein Molverhältnis von Wasser zu Gesamtmenge an Aminoalkyltrialkoxysilan von 10,5 bis 20 zu 1 bereitstellt, und
(c) Abdestillieren des bei der Reaktion gebildeten Alkylalkohols,

gelöst, wobei vorteilhaft ein Feststoffgehalt in einer so erhältlichen wässrigen Lösung (nachfolgend auch als wässrige Zusammensetzung oder kurz Zusammensetzung bezeichnet) von 30 bis 55 Gew.-%, bezogen auf die Zusammensetzung, bei gleichzeitig einer vergleichsweisen hohen Raum/Zeit-Ausbeute unter vergleichbaren apparativen Versuchsbedingungen und einem äußerst geringen VOC-Gehalt erzielt wird. So wurden insbesondere aufgrund der vorliegenden Arbeitsweise kürzere Dosierzeiten sowie kürzere und weniger intensive Rührzeiten ermöglicht und damit nicht zuletzt in energiesparender Weise eine deutliche Verbesserung der Wirtschaftlichkeit erzielt. Darüber hinaus treten bei erfindungsgemäßer Arbeitsweise auch keine Ausfällungen während des Herstellprozesses mehr auf, wodurch die Gefahr von Ablagerungen oder Verstopfungen in der Produktionsanlage gemindert und Stillstandzeiten deutlich verringert werden konnten.

Gegenstand der vorliegenden Erfindung ist somit ein Verfahren zur Herstellung von wässrigen Hydrolysaten von Aminoalkyltrialkoxysilanen durch

(a) Vorlegen von Wasser, optional Erwärmen,
(b) Zugeben von hydrolisierbaren Silanen bestehend aus mindestens einem Aminoalkyltrialkoxysilan in einer Menge, die ein Molverhältnis von Wasser zu Gesamtmenge an Aminoalkyltrialkoxysilan von 10,5 bis 20 zu 1 bereitstellt, und
(c) Abdestillieren des bei der Reaktion gebildeten Alkylalkohols,

wobei der Feststoffgehalt in einer so hergestellten wässrigen Zusammensetzung 30 bis 55 Gew.-%, bezogen auf die Zusammensetzung, beträgt, die wässrige Lösung einen Gesamtgehalt an freiem und gebundenen Alkylalkohol von kleiner gleich 1 Gew.-%, bezogen auf die Zusammensetzung, aufweist.

Aminoalkyltrialkoxysilane, die für den Einsatz gemäß der vorliegenden Erfindung geeignet sind, können ausgewählt sein aus Verbindungen der allgemeinen Formel

$$HR^1N - R^2 - Si(OR^3)_3,$$

worin

$R^1$ ausgewählt ist aus H oder einer $C_1$ - $C_4$ - Alkylgruppe;
$R^2$ ausgewählt ist aus einer bivalenten $C_1$ - $C_4$ - Alkylgruppe; und
$R^3$ ausgewählt ist aus einer $C_1$ - $C_3$- Alkylgruppe.

$R^1$ ist vorzugsweise Wasserstoff. $R^2$ ist vorzugsweise eine bivalente Propylgruppe $[-(CH_2)_3-]$. $R^3$ ist vorzugsweise aus Methyl und Ethyl ausgewählt.

Besonders bevorzugte Aminoalkyltrialkoxysilane sind 3-Aminopropyltriethoxysilan oder 3-Aminopropyltrimethoxysilan oder Mischungen davon, wobei 3-Aminopropyltriethoxysilan am meisten bevorzugt ist.

Bei dem erfindungsgemäßen Verfahren zur Herstellung von wässrigen Hydrolysaten von Aminotrialkoxysilanen wird das zur Hydrolyse benötigte Wasser vorgelegt und die hydrolysierbaren Silane, bestehend aus mindestens einem Aminoalkyltrialkoxysilan, zugegeben. Erfindungswesentlich ist hierbei, dass die Mengen an Aminoalkyltrialkoxysilan und Wasser so gewählt werden, dass nach vollständiger Zugabe der Aminoalkyltrialkoxysilane zu dem vorgelegten Wasser ein Molverhältnis von Wasser zu Gesamtmenge an Aminoalkyltrialkoxysilan von 10,5 bis 20 zu 1, insbesondere von 11 bis 18 zu 1, einschließlich 12:1, 13:1, 14:1, 15:1, 16:1, 17:1 bereitgestellt wird.

Zielsetzung der vorliegenden Erfindung ist, eine wässrige Lösung von Aminoalkyltrialkoxysilanen bereitzustellen, die im Wesentlichen frei von organischem Lösungsmittel, insbesondere des bei der Hydrolyse freigesetzten Alkohols, ist, und die auch nach evtl. Verdünnung mit Wasser im Wesentlichen keine organische Lösungsmittel, insbesondere den durch die Hydrolyse gebildete Alkohol, freisetzt. "Im Wesentlichen frei" im Sinne der vorliegenden Erfindung bedeutet, dass die erhaltene wässrige Lösung einen Gehalt an organischen Lösungsmitteln, insbesondere einen Gesamtgehalt an freiem Alkylalkohol und gebundenem Alkylalkohol, der durch Hydrolyse freigesetzt werden könnte, von kleiner gleich 1 Gew.-%, bezogen auf die Zusammensetzung, vorzugsweise von kleiner gleich 0,7 Gew.-% bis hin zur Nachweisgrenze, aufweist, gemessen nach Hydrolyse einer Probe der wässrigen Lösungen mit Schwefelsäure und nachfolgender Neutralisation mit wässriger Sodalösung mittels Gaschromatographie (genaue Vorschrift zur Messung des Gesamtgehalts an Alkylalkohol siehe Beispiele).

Es hat sich überraschenderweise herausgestellt, dass bei dem erfindungsgemäßen Verfahren das Ausgangsaminoalkyltrialkoxysilan bereits nach verhältnismäßig kurzen Zugabe- bzw. Dosier- oder Rührzeiten in Schritt (b) des Verfahrens im Wesentlichen vollständig hydrolysiert ist und keine Ausfällungen auftreten. Insbesondere ist nach Beendigung der Zugabe des Aminoalkyltrialkoxysilans keine weitere Reaktionszeit erforderlich, um die oben angegebenen Werte an Gesamtalkylalkohol zu erreichen und es kann unmittelbar nach Beendigung der Zugabe mit dem Abdestillieren des bei der Reaktion gebildeten Alkylalkohols begonnen werden (Schritt (c)). Dadurch lassen sich im Vergleich zum Stand der Technik unter vergleichbaren apparativen Bedingungen deutlich verbesserte Raum/Zeit-Ausbeuten (RZA) bei dem erfindungsgemäßen Verfahren erzielen, wodurch die Wirtschaftlichkeit eines Verfahrens zur Herstellung von wässrigen Hydrolysaten von Aminoalkyltrialkoxysilanen deutlich verbessert werden kann, ohne dass sich dadurch die Produkteigenschaften der erhaltenen wässrigen Lösungen verschlechtern.

So kann die Zeitspanne zwischen Beendigung der Zugabe der Aminoalkyltrialkoxysilane und Beginn des Abdestillierens des Alkylalkohols weniger als 5 Minuten nach Erreichen der bevorzugten bzw. erforderlichen Destillationsbedingungen, d. h. insbesondere nach Einstellen von Druck und Temperatur in der Reaktions-/Destillationseinheit, vorzugsweise weniger als 3 Minuten, besonders bevorzugt weniger als 1 Minute betragen, insbesondere ohne weitere Produktionsverzögerung, [nachfolgend auch kurz min = Minute(n) sowie h= Stunde(n)].

Vorzugsweise beträgt die Reaktorinnentemperatur bei Beginn der Zugabe der Aminoalkyltrialkoxysilane in Schritt (b) 25 °C bis 50 °C, besonders bevorzugt 30 °C bis 45 °C. Da die Hydrolysereaktion von Aminoalkyltrialkoxysilan exotherm ist, erwärmt sich die Reaktionsmischung bei Zugabe der Aminoalkyltrialkoxysilane zu dem vorgelegten Wasser. Daher hat es sich als vorteilhaft erwiesen, die Zugabedauer in Schritt (b) so einzustellen, dass die Reaktorinnentemperatur bei Beendigung der Zugabe der Aminoalkyltrialkoxysilane in Schritt (b) 50 °C bis 80 °C, vorzugsweise 55 °C bis 70 °C, besonders bevorzugt 55 °C bis 65 °C beträgt. Die Dauer der Zugabe des Aminoalkyltrialkoxysilans in Schritt (b) beträgt daher vorzugsweise 20 bis 240 min, besonders bevorzugt 30 bis 120 min, insbesondere bevorzugt 35 bis 90 min und am meisten bevorzugt 40 bis 60 min.

Nach Beendigung der Hydrolysereaktion wird gemäß Schritt (c) des erfindungsgemäßen Verfahrens der bei der Reaktion gebildete Alkylalkohol abdestilliert. Nach Abdestillieren des gebildeten Alkylalkohols sollte der Gehalt an freiem Alkylalkohol kleiner gleich 1 Gew.-%, vorzugsweise kleiner gleich als 0,7 Gew.-%, betragen, gemessen mittels Gaschromatographie (genaue Vorschrift zu Messung des freien Alkylalkohols siehe Beispiele). Vorzugsweise wird der durch die Hydrolyse entstehende Alkohol unter vermindertem Druck abdestilliert. Ein geeigneter Druckbereich für die Vakuumdestillation beträgt 80 bis 400 mbar, vorzugsweise 100 bis 350 mbar bei einer Temperatur von 30 °C bis 70 °C vorzugsweise 35°C bis 60 °C. Zur Einstellung der gewünschten Konzentration der als Reaktionsprodukt erhaltenen wässrigen Lösung bzw. zur Einstellung der Viskosität der wässrigen Lösung kann die Menge an abdestilliertem Alkylalkohol, bzw. Gemisch aus Wasser und Alkylalkohol, zumindest teilweise durch Wasser ersetzt werden.

Durch das erfindungsgemäße Verfahren wird eine wässrige Lösung eines Hydrolysats von hydrolysierbaren Silanen, bestehend aus mindestens einem Aminoalkyltrialkoxysilan, erhalten, die einen hohen Feststoffanteil im Bereich von 45 bis 55 Gew.-%, gemessen nach DIN 38409-H1-1 aufweist.

Bei den erfindungsgemäßen wässrigen Lösungen handelt es sich um optisch klare, im Wesentlichen farblose, wässrige Lösungen. Insbesondere hat es sich überraschenderweise gezeigt, dass die erfindungsgemäßen Lösungen auch nach längerer Lagerung farblos sind.

Somit sind die erfindungsgemäßen Lösungen insbesondere für Anwendungen geeignet, bei denen es unerwünscht ist, wenn durch wässrige Lösungen von Hydrolysaten von Aminoalkyltrialkoxysilan die Farbe des Systems verändert wird. Dies betrifft insbesondere die Verwendung der erfindungsgemäßen wässrigen Lösung in Harz- oder Lacksystemen, insbesondere farblosen Harz- und Lacksystemen.

Weiterhin sind die erfindungsgemäßen Lösungen optisch klar und weisen vorzugsweise einen Trübwert von 5 FNU oder weniger, besonders bevorzugt 3 FNU oder weniger, insbesondere 2 FNU oder weniger und am meisten bevorzugt 1 FNU oder weniger, gemessen nach ISO 7027, auf.

Die erfindungsgemäßen wässrigen Lösungen fallen bei dem erfindungsgemäßen Verfahren vorteilhaft mit einem Feststoffgehalt von 30 bis 55 Gew.-%, bezogen auf die Zusammensetzung, vorzugsweise von 40 bis 55 Gew.-

%, besonders bevorzugt von 44 bis 54 Gew.-%, insbesondere 50 bis 53 Gew.-% an.

Aufgrund der hohen Feststoffkonzentration haben die erfindungsgemäßen wässrigen Lösungen zahlreiche Vorteile gegenüber den bekannten kommerziellen Produkten. So weisen die wässrigen Lösungen einen Gefrierpunkt im Bereich von -7 °C bis -18 °C, vorzugsweise von -9 °C bis - 17 °C, besonders bevorzugt zwischen -10 °C und -16 °C auf, bei einem Druck von 1 atm. Das heißt, die erfindungsgemäßen Lösungen sind deutlich frostresistenter und müssen nicht bei entsprechend niedrigen Temperaturen im Wärmetransport transportiert werden, wodurch die Energiekosten noch einmal reduziert werden können.

Weiterhin reduzieren sich durch den hohen Feststoffgehalt die Transportkosten, bezogen auf die Aminoalkyltrialkoxysilan-Hydrolysatmasse, deutlich. Weiterhin eignen sich die erfindungsgemäßen wässrigen Lösungen besonders zur Herstellung von Konzentraten, da im Vergleich zu den kommerziell erhältlichen Produkten deutlich weniger Wasser eingebracht wird.

Im Allgemeinen kann man das erfindungsgemäße Verfahren wie folgt durchführen:

In einer geeigneten Apparatur, beispielsweise bestehend aus Reaktor mit Dosier- und Entnahmevorrichtungen, Heiz-/Kühlvorrichtung einschließlich Mess- und Regelungseinheit sowie Rückflusskühler, Mischeinheit, Destillationseinheit einschließlich Druckminderungsvorrichtung, legt man das Wasser vor, erwärmt optional auf die gewünschte Temperatur, vorzugsweise auf eine Temperatur von 25 bis 50 °C, insbesondere auf eine Temperatur im Bereich von 30 bis 45 °C, und dosiert unter Durchmischung das Aminoalkyltrialkoxysilan in einer Menge, die dem Molverhältnis von Wasser zu Gesamtmenge an Aminoalkyltrialkoxysilan von 10,5 bis 20 zu 1 entspricht. Dabei arbeitet man geeigneterweise unter Schutzgas, beispielsweise unter Stickstoff. Anschließend destilliert man den bei der Reaktion gebildeten Alkylalkohol ab und gewinnt das Verfahrensprodukt.

Erfindungsgemäß erhaltene wässrigen Lösungen finden -optional auch nach Verdünnung, beispielsweise mit VE-Wasser- vorteilhaft Verwendung für die Beschichtung von Glasfasern oder Steinwolle, für die Silanierung von Füllstoffen, als Haftvermittler, insbesondere für die Verbesserung der Haftung organischer Polymerer an anorganische Oberflächen, zur Herstellung von Korrosionsschutzkonzentraten, zur Modifizierung von Harzsystemen insbesondere Aminoplast-Formaldehyd-Harze oder Phenolharze, für die Verbesserung rheologischer Eigenschaften insbesondere von wässrigen Polymerdispersionen und Emulsionen, als Vernetzer, als Modifiziermittel für Farben und Lacke.

Die vorliegende Erfindung wird nachfolgend unter Bezugnahme auf die beigefügten Ausführungsbeispiele näher erläutert, ohne den Gegenstand der Erfindung darauf zu beschränken:

**Beispiele:**

Die Messmethoden zur Charakterisierung der Eigenschaften der wässrigen Lösungen, die nach dem erfindungsgemäßen Verfahren hergestellt werden, wie sie in der Beschreibung, den Beispielen und den Ansprüchen angegeben sind, wurden wie folgt durchgeführt:

| | |
|---|---|
| Feststoff [Gew.-%] | Der Feststoffgehalt wurde gemäß DIN 38409-H1-1 bestimmt. |
| $SiO_2$ [Gew.-%] | Die $SiO_2$-Bestimmung wurde wie nachfolgend beschrieben durchgeführt: 1 g einer Probe wurde in ein 250ml Becherglas eingewogen. Eine Kjeldahltablette (Merck Nr. 15348) und 20 ml konzentrierte Schwefelsäure wurde zugesetzt. Die Lösung wurde langsam erwärmt bis sich $SO_3$-Dämpfe bilden. Nach Abkühlen wurde die Lösung vorsichtig mit destilliertem Wasser auf 200ml verdünnt. Die ausgefallene Kieselsäure wurde durch ein Weißbandfilterpapier filtriert. Der Filterrückstand wurde mit destilliertem Wasser bis zur Neutralität des Filtrats (pH > 4) gewaschen. Das Filterpapier wurde in einem Platintiegel getrocknet und verascht. Der Rückstand wurde bei 800 °C verbrannt und gewogen. Nach Behandlung mit konzentrierter Flusssäure wurde der Rückstand wieder bei 800 °C verbrannt und gewogen. $SiO_2$ [Gew.-%] = 100m/E m = Gewichtsdifferenz vor und nach der Behandlung mit Flusssäure in g E = Gewicht der Probe in g. |
| Viskosität (20 °C) [mPas] | Bestimmung gemäß DIN 53015 |
| Dichte (20 °C) [g/ml] | Bestimmung gemäß DIN 51757 |
| Brechzahl (20 °C) | Bestimmung DIN 51423 |

(fortgesetzt)

| Trübung [FNU] | Bestimmung gemäß ISO 7027 |
|---|---|
| Gesamtalkohol (Alkohol, n. Hydrolyse) [Gew.-%] | Die Gesamtalkohol-Bestimmung wurde wie nachfolgend beschrieben durchgeführt: 5 g einer Probe wurden in einen Kolben eingewogen und mit 25 ml Schwefelsäure (20 %) hydrolysiert. Nach Zugabe von 75 ml destilliertem Wasser wurde die Lösung mit Sodalösung (20 %) neutralisiert und anschließend einer Wasserdampfdestillation unterzogen. Das Destillat wurde in einem 250 ml Kolben gesammelt. Entsprechend des zu erwartenden Alkoholgehalts wurden bis zu 3 ml sek-Butanol zugegeben und der Kolben bis zur Markierung mit destilliertem Wasser aufgefüllt. Die Alkohole wurden quantitative mittels Gaschromatographie unter Verwendung einer Kapillarsäule mit FID und entsprechender Datenverarbeitung (HP 7820 mit OpenLab) analysiert. Lösungen der zu erwartenden Alkohole und von sek-Butanol wurden zu Kalibrierungszwecken verwendet. Das sek-Butanol diente als interner Standard. Der so bestimmte Gesamtalkoholgehalt in der Probe beinhaltet den Anteil an freiem und in Form von hydrolysierbaren Alkoxygruppen gebundenen Alkohol. |
| Freier Alkohol (Ethanol) [Gew.-%] | Die Bestimmung des Anteils an freiem Alkohol wurde wie nachfolgend beschrieben durchgeführt: 2 g einer Probe und 50 mg von 2-Butanol als interner Standard werden in 10 g VE-Wasser gelöst. Diese Lösung wird mittels Gaschromatographie unter Verwendung einer Kapillarsäule mit TCD und entsprechender Datenverarbeitung (HP 7820 mit OpenLab) analysiert. Die Kalibrierung erfolgt mit einer Lösung von 50 mg des relevanten Alkohols und 50 mg des verwendeten internen Standards in 10 g des verwendeten Lösungsmittels. |

Die in Beispielen angegebene Raum-Zeit-Ausbeute (RZA) wurde wie folgt berechnet:

$$RZA = \frac{\text{Ausbeute gerechnet als Silicium (Si) in [g]}^{1)}}{\sum (\text{Volumen Edukte}) \, [l]^{\,2)} \times \text{Herstellzeit } [h]^{3)}}$$

[1] Ausbeute (Si) = Ausbeute [g] x w(Si) in % x 0,01 Dabei wurde "(Si)" bzw. "w(Si)" auf der Grundlage der jeweiligen $SiO_2$-Bestimmung berechnet.

[2] Als Edukte werden die vorgelegten Wasser- bzw. 3-Aminopropyltriethoxysilan- (AMEO) Mengen und die zudosierten AMEO- bzw. Wassermengen bezeichnet. Die Dichte für die eingesetzten Edukte wurde vereinfacht mit 1,0 g/$cm^3$ angenommen. Damit berechnet sich das Volumen Edukte aus dem Quotient $\Sigma(m_{Edukte})$ und der Dichte von 1,0 g/$cm^3$.

[3] Herstellzeit = Zeit ab Start der Reaktion (Zutropfen von AMEO bzw. Wasser) bis Destillationsende

**Beispiel 1:**

In einen 2 l Vierhalskolben mit KPG-Rührer, Tropftrichter, Destillationsbrücke, Intensivkühler, Öldrehschieberpumpe, Sumpfthermometer, Kopfthermometer Ölbad mit Temperaturregelung wurden 800,0 g (44,4 Mol) VE-Wasser unter $N_2$-Überlagerung vorgelegt und mit dem Ölbad auf eine Sumpftemperatur von 40,7 °C erwärmt. Nach dem Entfernen des Ölbades wurde ohne weiteres Erwärmen innerhalb von 44 min 800,0 g (3,61 Mol) Dynasylan® AMEO zugetropft. Die Sumpftemperatur stieg dabei bis auf 57,5 °C an. Es entstand dabei für ca. 6 min eine minimale Trübung, danach war der Sumpf farblos klar. Nach beendetem Zutropfen wurde sofort Vakuum angelegt. Bei einem Vakuum von 304 bis 122 mbar, Badtemperatur 105°C bis 115 °C, einer Sumpftemperatur von 46,3 °C bis 50,7 °C, Kopftemperatur von 44,4 °C bis 49,4 °C wurde innerhalb von 2,3 h 842,7 g Ethanol/Wasser abdestilliert (Hydrolysealkohol + 73 %) Nachdem ca. 500 g abdestilliert waren, wurde begonnen 579,2 g VE-Wasser bis zum Ende der Destillation zu zutropfen. Während der Reaktion und Destillation kam es zu keiner Ausfällung oder starkem Viskositätsanstieg. Nach beendeter Destillation wurde mit Stickstoff belüftet.

Die Ausbeute betrug 1320,22 g farblos, klares Produkt.

| | |
|---|---|
| Die Raum-Zeit-Ausbeute betrug | 21,3 [g (Si) / (lxh)]. |
| Feststoffgehalt | 30,7 Gew.-% |
| Gesamtalkohol | 0,7 Gew.-% |

Die Analysendaten des erhaltenen Produkts sind in Tabelle 1 nochmals zusammenfassend gegenübergestellt.

**Vergleichsbeispiel 1:** (vgl. Example 2 aus US 6,512,132)

In einen 2 l Vierhalskolben mit KPG-Rührer, Tropftrichter, Destillationsbrücke, Intensivkühler, Öldrehschieberpumpe, Sumpfthermometer, Kopfthermometer Ölbad mit Temperaturregelung wurden 800,0 g (3,61 Mol) Dynasylan® AMEO unter $N_2$-Überlagerung vorgelegt und mit dem Ölbad auf eine Sumpftemperatur von 41,4 °C erwärmt. Nach dem Entfernen des Ölbades, wurde ohne weiteres Erwärmen innerhalb von 2 h 800,0 g (44,4 Mol) VE-Wasser zugetropft. Die Sumpftemperatur stieg dabei bis auf 65,4 °C an und es war eine starke Exothermie erkennbar. Es entstand dabei über einen Zeitraum von ca. 8 min. eine starke Ausfällung, mit zum Teil großen, weißen Flocken. Anschließend war der Sumpf farblos klar. Nach beendetem Zutropfen, wurde sofort Vakuum angelegt. Bei einem Vakuum von 204 bis 198 mbar, Badtemperatur 109 °C bis 111 °C, einer Sumpftemperatur von 46,8 °C bis 59,9 °C, Kopftemperatur von 42,8 °C bis 51,3 °C wurde innerhalb von 2 h 701,6 g Ethanol/Wasser abdestilliert (Hydrolysealkohol +40 %). Nach dem Abkühlen auf ca. 38 °C wurde mit Stickstoff belüftet und das Produkt innerhalb von ca. 15 min mit 435,6 g VE-Wasser verdünnt und so auf einen Wirkstoffgehalt von 60 % eingestellt.

Die Ausbeute betrug 1324,4 g farblos, klares Produkt.

| | |
|---|---|
| Die Raum-Zeit-Ausbeute betrug | 11,2 [g (Si) / (lxh)]. |
| Feststoffgehalt | 30,8 Gew.-% |
| Gesamtalkohol | 1,4 Gew.-% |

Die Analysendaten des erhaltenen Produkts sind in Tabelle 1 nochmals zusammenfassend gegenübergestellt.

**Beispiel 2:**

In einen 2 l Vierhalskolben mit KPG-Rührer, Tropftrichter, Destillationsbrücke, Intensivkühler, Öldrehschieberpumpe, Sumpfthermometer, Kopfthermometer Ölbad mit Temperaturregelung wurden 800,0 g (44,4 Mol) VE-Wasser unter $N_2$-Überlagerung vorgelegt und mit dem Ölbad auf eine Sumpftemperatur von 40,2 °C erwärmt. Nach dem Entfernen des Ölbades wurde ohne weiteres Erwärmen, innerhalb von 40 min 800,0 g (3,61 Mol) Dynasylan® AMEO zugetropft. Die Sumpftemperatur stieg dabei bis auf 58,2 °C an. Es entstand dabei eine minimale Trübung, die nach beendetem Zutropfen verschwand, der Sumpf war farblos, klar. Danach wurde sofort Vakuum angelegt. Bei einem Vakuum von 326 bis 101 mbar, Badtemperatur 108 °C bis 110 °C, einer Sumpftemperatur von 48,4 °C bis 52,9 °C, Kopftemperatur von 46,7 °C bis 49,6 °C wurde innerhalb von 2,4 h 872,0 g Ethanol/Wasser abdestilliert (Hydrolysealkohol + 73 %). Nachdem ca. 700 g abdestilliert waren, wurde innerhalb von 10 min 46,9 g VE-Wasser zum verdünnen zugetropft. Der Sumpf war gegen Ende der Destillation viskos, jedoch noch gut zu rühren. Nach dem Abkühlen, wurde die Anlage mit Stickstoff belüftet.

Die Ausbeute betrug 762,9 g farblos, klares Produkt.

| | |
|---|---|
| Die Raum-Zeit-Ausbeute betrug | 21,1 [g (Si) / (lxh)]. |
| Feststoffgehalt | 52,8 Gew.-% |
| Gesamtalkohol | 0,2 Gew.-% |

Die Analysendaten des erhaltenen Produkts sind in Tabelle 1 nochmals zusammenfassend gegenübergestellt.

**Vergleichsbeispiel 2:** (vgl. Example 1 aus US 6,512,132)

In einen 2 l Vierhalskolben mit KPG-Rührer, Tropftrichter, Destillationsbrücke, Intensivkühler, Öldrehschieberpumpe, Sumpfthermometer, Kopfthermometer Ölbad mit Temperaturregelung wurden 1000,0 g (4,52 Mol) Dynasylan® AMEO unter $N_2$-Überlagerung vorgelegt und mit dem Ölbad auf eine Sumpftemperatur von 40,1 °C erwärmt. Nach dem Entfernen des Ölbades, wurde ohne weiteres Erwärmen innerhalb von 1,25 h 500,0 g (27,8 Mol) VE-Wasser zugetropft. Die Sumpftemperatur stieg dabei bis auf 65,5 °C an und es war eine starke Exothermie erkennbar. Es entstand dabei über einen Zeitraum von ca. 18 min. eine starke Ausfällung, mit zum Teil großen, weißen Flocken. Nach beendetem Zutropfen, wurde sofort Vakuum angelegt. Bei einem Vakuum von 216 bis 205 mbar, Badtemperatur 108 °C bis 113 °C, einer Sumpftemperatur von 46,9 °C bis 52,9 °C, Kopftemperatur von 42,6 °C bis 48,3 °C wurde innerhalb von 1,65 h 703,4 g Ethanol/Wasser abdestilliert (Hydrolysealkohol +20 %). Während der Destillation kam es zu einem sehr starken Viskositätsanstieg. Dabei war der Sumpf nicht mehr rührfähig. Der Sumpf war fest (Wirkstoffgehalt 125,5 %).

Es wurde sofort mit Stickstoff belüftet und der Sumpf mit 250,4 g VE-Wasser versetzt. Um den Feststoff wieder in Lösung zu bekommen, wurde der Kolben 5,25 h auf der Schüttelmaschine RO 10 geschüttelt. Danach war der Sumpf fast vollständig wieder in Lösung. Lediglich am Rührblatt war noch etwas Feststoff vorhanden.

Die Ausbeute betrug 1037,6 g farblos, klares Produkt.

Die Raum-Zeit-Ausbeute beträgt 11,7 g Si / (lxh).

| | |
|---|---|
| Die Raum-Zeit-Ausbeute betrug | 11,7 [g (Si) / (lxh)]. |
| Feststoffgehalt | 48,6 Gew.-% |

| | |
|---|---|
| Gesamtalkohol | 6,5 Gew.-% |

Die Analysendaten des erhaltenen Produkts sind in Tabelle 1 nochmals zusammenfassend gegenübergestellt.

**Beispiel 3:**

In einen 8 l Vierhalsdoppelmantelkolben mit KPG-Rührer, Tropftrichter, Destillationsbrücke, Kolonne mit 4 Sulzerpackungen EX L = 5 cm, B = 4 cm (theoretische Böden bei mittlerer Belastung 13 - 20) Intensivkühler, Öldrehschieberpumpe, Sumpfthermometer, Kopfthermometer, Thermostat, Dosierpumpe Prominent Gamma 4 wurden 3401,8 g (188,8 Mol) VE-Wasser unter $N_2$-Überlagerung vorgelegt und mit dem Thermostaten auf eine Sumpftemperatur von 40,0 °C erwärmt. Bei dieser Sumpftemperatur (Badtemp. 60 °C) wurde dann innerhalb 1 h 3400,1 g (15,4 Mol) Dynasylan® AMEO über eine Dosierpumpe zudosiert. Die Sumpftemperatur stieg dabei bis auf 63,9 °C an. Der Sumpf blieb hierbei farblos, klar. Danach wurde sofort Vakuum angelegt. Bei einem Vakuum von 106 bis 105 mbar, Thermostattemperatur 96,8 °C bis 105 °C, einer Sumpftemperatur von 37,8 °C bis 47,7 °C, Kopftemperatur von 36,6 °C bis 46,9 °C wurde innerhalb von 4,75 h 3719,3 g Ethanol/Wasser abdestilliert (Hydrolysealkohol + 75 %). Nachdem ca. 1348,71 g abdestilliert waren, wurde während der Destillation innerhalb von 2,5 h insgesamt 782,9 g VE-Wasser zum verdünnen zugetropft. Der Sumpf war während und gegen Ende der Destillation farblos, klar, nicht viskos. Es gab keinerlei Ausfällungen und Ablagerungen am Kolbenrand. Nach dem Abkühlen wurde die Anlage mit Stickstoff belüftet.

Die Ausbeute betrug 3897,2 g farblos klares Produkt.

| | |
|---|---|
| Die Raum-Zeit-Ausbeute betrug | 12,0 [g (Si) / (lxh)]. |
| Feststoffgehalt | 44,7 Gew.-% |
| Gesamtalkohol | 0,3 Gew.-% |

Die Analysendaten des erhaltenen Produkts sind in Tabelle 1 nochmals zusammenfassend gegenübergestellt.

**Vergleichsbeispiel 3:**

In einen 8 l Vierhalsdoppelmantelkolben mit KPG-Rührer, Tropftrichter, Destillationsbrücke, Kolonne mit 4 Sulzerpackungen EX L = 5 cm, B = 4 cm (theoretische Böden bei mittlerer Belastung 13 bis 20) Intensivkühler, Öldrehschieberpumpe, Sumpfthermometer, Kopfthermometer , Thermostat, Dosierpumpe Prominent Gamma 4 wurden 3406,5 g (15,4 Mol) Dynasylan® AMEO unter $N_2$-Überlagerung vorgelegt und mit dem Thermostaten auf eine Sumpftemperatur von 39,9 °C erwärmt. Bei dieser Sumpftemperatur (Badtemp. 60 °C) wurde dann innerhalb 2,75 h 3400,5 g (188,8 Mol) VE-Wasser über eine Dosierpumpe zudosiert. Die Sumpftemperatur stieg dabei bis auf 72,7 °C an. Es war eine sehr starke Exothermie erkennbar. Nachdem ca. 225 ml VE-Wasser zudosiert waren, bildeten sich weiße Flocken und Ablagerungen am Kolbenrand. Der Sumpf war milchig weiß. Nach weiteren 7 min (insgesamt 300 ml VE-Wasser) waren die Flocken und Trübungen wieder in Lösung, der Sumpf blieb danach farblos, klar. Nachdem alles VE-Wasser zudosiert war, wurde sofort Vakuum angelegt. Bei einem Vakuum von 107 bis 100 mbar, Thermostattemperatur 81 °C bis 105 °C, einer Sumpftemperatur von 37,8 °C bis 47,7 °C, Kopftemperatur von 35,2 °C bis 50,9 °C wurde innerhalb von 5,6 h 3820,3 g Ethanol/Wasser ab destilliert (Vorlage + Kühlfalle) (Hydrolysealkohol + 80 %). Nachdem ca. 1632,4 g ab destilliert waren, traten am Kolbenrand gelartige Ablagerungen auf, die während der Destillation weiter anreicherten. Der Sumpf war nach Ende der Destillation viskos, farblos, klar (Viskosität -3000 mPa*s Messung aus Versuch IL/V180/12-43). Nach Ende der Destillation wurde der Sumpf innerhalb von 5 min mit 884,45 g VE-Wasser auf einen Wirkstoffgehalt von 88 % verdünnt und das ganz ca. 30 min bei 48,5 °C bis 41,3 °C nachgerührt. Das Gel vom Kolbenrand war zu diesem Zeitpunkt noch nicht vollständig gelöst. Nach dem Abkühlen wurde die Anlage mit Stickstoff belüftet.

Die Ausbeute betrug 3761,5 g farblos, klares Produkt.

| Die Raum-Zeit-Ausbeute betrug | 6,3 | [g (Si) / (lxh)]. |
| Feststoffgehalt | 45,0 | Gew.-% |
| Gesamtalkohol | 0,1 | Gew.-% |

Die Analysendaten des erhaltenen Produkts sind in Tabelle 1 nochmals zusammenfassend gegenübergestellt.

**Vergleichsbeispiel 4:** (vgl. Beispiel 1 aus EP1031593A2)

In einen 4 l Vierhalskolben mit KPG-Rührer, Tropftrichter, Destillationsbrücke, Intensivkühler, Öldrehschieberpumpe, Sumpfthermometer, Kopfthermometer Ölbad mit Temperaturregelung wurden 1250,0 g (69,4 Mol) VE-Wasser unter $N_2$-Überlagerung vorgelegt. Anschließend wurde innerhalb von 35 Minuten ein Gemisch aus 480,0g (2,17 Mol) Dynasylan® AMEO und 120,0g (0,46 Mol) AMEO-Hochsieder über die Dosiervorrichtung zu getropft (AMEO Hochsieder ist ein Gemisch aus Dynasylan® AMEO und ca. 20% Bis-AMEO). Dabei steigt die Sumpftemperatur von Raumtemperatur auf 50°C an. Der Ansatz wurde 3h bei 50°C gerührt. Bei einem Absolutdruck von 130 mbar bis 100 mbar werden bei einer Sumpftemperatur von 39°C bis 41°C 450 g Ethanol / Wasser Gemisch in ca. 1,3 h ab destilliert. Das Endprodukt wird am Ende mit Wasser auf ein Gewicht von 1500g eingestellt.
Die Ausbeute betrug 1500,0 g farbloses leicht trübes Produkt.

| Die Raum-Zeit-Ausbeute betrug | 7,2 | [g (Si) / (lxh)]. |
| Feststoffgehalt | 20,1 | Gew.-% |
| Gesamtalkohol | nicht | bestimmt |

Die Analysendaten des erhaltenen Produkts sind in Tabelle 1 nochmals zusammenfassend gegenübergestellt.

Tabelle 1: : Gegenüberstellung der Analysendaten der erhaltenen Produkte aus den Beispielen

| | B 1 | VB 1 | B 2 | VB 2 | B 3 | VB 3 | VB 4 |
|---|---|---|---|---|---|---|---|
| Molares Verhältnis der Einsatzstoffe Wasser zu "Silan-Komponente" | 12,3 : 1 | 12,3 : 1 | 12,3 : 1 | 6,2 : 1 | 12,2 : 1 | 12,3 : 1 | 26,4 : 1 |
| Feststoffgehalt im Produkt [Gew.-%] | 30,7 | 30,8 | 52,8 | 48,6 | 44,7 | 45,0 | 20,1 |
| $SiO_2$-Gehalt im Produkt [Gew.-%] | 16,4 | 16,4 | 28,6 | 26,2 | 21,3 | 21,8 | 9,3 |
| Viskosität (20 °C) [mPas] | 10,6 | 11,0 | 550 | 267 | 80,3 | 92 | n.d. |
| Trübung [FNU] | 0,74 | 0,9 | 1,9 | 0,5 | 0,3 | 1,3 | 4 |
| Gesamt-Ethanol (nach Hydrolyse) [Gew.-%] | 0,7 | 1,4 | 0,2 | 6,5 | 0,3 | 0,1 | n.d.. |
| Freies Ethanol im Produkt [Gew.-%] | 0,7 | 1,4 | 0,2 | 5,9 | 0,3 | 0,1 | n.d. |
| Farbzahl [mg Pt-Co/l] | <5 | <5 | <5 | <5 | <5 | <5 | n.d. |
| Raum-Zeit-Ausbeute [g (Si) / (lxh)] | 21,3 | 11,2 | 21,1 | 11,7 | 12,0 | 6,3 | 7,2 |

Zusammenfassung der Ergebnisse aus den Versuchen:

Die Beispiele B1, VB1, B2 und VB2, vgl. Tabelle 1, wurden unter vergleichbaren apparativen Bedingungen durchgeführt. Bei erfindungsgemäßer Verfahrensweise liegt die RZA und damit die Wirtschaftlichkeit gegenüber der gemäß US 6,512,132 deutlich höher. Ferner fällt in VB2 ein Produkt mit einem deutlich höheren Gesamtethanol-Gehalt an.
Auch können die Beispiele B3 und VB3 mit Hinblick auf die apparativen Voraussetzungen für einen Vergleich der RZA dienen. So weist das erfindungsgemäß durchgeführte Beispiel 3 gegenüber Vergleichsbeispiel 3 eine deutliche bessere Wirtschaftlichkeit auf, wie anhand der RZA belegt. Auch liegt der Trübungswert im Produkt aus VB3 deutlich über dem aus B3.
Vergleichsbeispiel 4 (VB4) entspricht der Lehre gemäß Beispiel 1 aus EP1031593A2), dabei wird nur ein Feststoffgehalt von 20 Gew.-% erzielt.
Des Weiteren sind die Dosier- und/oder Rührzeiten in VB1, VB2, VB3 oder VB4 deutlich länger als in den erfindungsgemäßen Beispielen B1, B2 und B3.

Darüber hinaus weisen erfindungsgemäße Produkte aus B1, B2 und B2 einen hohen Feststoffgehalt und eine ökologisch vorteilhaft niedrigen VOC-Gehalt (in Form von Gesamt-Ethanol) auf, was ein Maß für die Vollständigkeit der Hydrolysereaktion und damit nicht zuletzt - neben der besonderen Wirtschaftlichkeit des erfindungsgemäßen Verfahrens - auch ein Maß für seine Qualität darstellt.

**Patentansprüche**

1. Verfahren zur Herstellung von wässrigen Hydrolysaten von Aminoalkyltrialkoxysilanen durch

   (a) Vorlegen von Wasser, optional Erwärmen,
   (b) Zugeben von hydrolisierbaren Silanen bestehend aus mindestens einem Aminoalkyltrialkoxysilan in einer Menge, die ein Molverhältnis von Wasser zu Gesamtmenge an Aminoalkyltrialkoxysilan von 10,5 bis 20 zu 1 bereitstellt, und
   (c) Abdestillieren des bei der Reaktion gebildeten Alkylalkohols,

   wobei der Feststoffgehalt in einer so hergestellten wässrigen Zusammensetzung 30 bis 55 Gew.-%, bezogen auf die Zusammensetzung, beträgt, die wässrige Lösung einen Gesamtgehalt an freiem und gebundenen Alkylalkohol von kleiner gleich 1 Gew.-%, bezogen auf die Zusammensetzung, aufweist.

2. Verfahren nach Anspruch 1, wobei die Aminoalkyltrialkoxysilane ausgewählt sind aus Verbindungen der allgemeinen Formel

$$HR^1N - R^2 - Si(OR^3)_3,$$

   worin

   $R^1$ ausgewählt ist aus H oder einer $C_1$ - $C_4$ - Alkylgruppe;
   $R^2$ ausgewählt ist aus einer bivalenten $C_1$ - $C_4$ - Alkylgruppe; und
   $R^3$ ausgewählt ist aus einer $C_1$ - $C_3$- Alkylgruppe.

3. Verfahren nach Anspruch 2, wobei $R^1$ H ist.

4. Verfahren nach einem der Ansprüche 2 oder 3, wobei $R^2$ eine bivalente Propylgruppe $[-(CH_2)_3-]$ ist und $R^3$ aus Methyl und Ethyl ausgewählt ist.

5. Verfahren nach einem der Ansprüche 1 bis 4, wobei das Aminoalkyltrialkoxysilan aus 3-Aminopropyltriethoxysilan oder 3-Aminopropyltrimethoxysilan oder Mischungen davon ausgewählt ist, insbesondere ist als Aminoalkyltrialkoxysilan das 3-Aminopropyltriethoxysilan bevorzugt.

6. Verfahren nach einem der vorstehenden Ansprüche, wobei der Feststoffgehalt in einer so hergestellten wässrigen Zusammensetzung 40 bis 55 Gew.-%, insbesondere 50 bis 53 Gew.-%, bezogen auf die Zusammensetzung, beträgt.

7. Verfahren nach einem der vorstehenden Ansprüche, wobei das Molverhältnis von Wasser zu Gesamtmenge an Aminoalkyltrialkoxysilan 11 bis 18 zu 1 beträgt, hier eingeschlossen die molaren Verhältnisse 12:1, 13:1, 14:1, 15:1, 16:1, 17:1.

8. Verfahren nach einem der vorstehenden Ansprüche, wobei die Dauer der Aminoalkyltrialkoxysilan-Zugabe in Schritt (b) 20 bis 240 Minuten, vorzugsweise 30 bis 120 Minuten, besonders bevorzugt 35 bis 90 Minuten, am meisten bevorzugt 40 bis 60 Minuten beträgt.

9. Verfahren nach einem der vorstehenden Ansprüche, wobei die Reaktorinnentemperatur bei Beginn der Zugabe der Aminoalkyltrialkoxysilane in Schritt (b) 25 °C bis 50 °C, vorzugsweise 30 °C bis 45 °C beträgt und die Reaktorinnentemperatur bei Beendigung der Zugabe der Aminoalkyltrialkoxysilane in Schritt (b) 50 °C bis 80 °C, vorzugsweise 55 °C bis 70 °C, besonders bevorzugt 55 °C bis 65 °C beträgt.

10. Verfahren nach einem der vorstehenden Ansprüche, wobei mit dem Abdestillieren des Alkylalkohols unmittelbar nach Beendigung der Zugabe der Aminoalkyltrialkoxysilane begonnen wird.

11. Verfahren nach einem der vorstehenden Ansprüche, wobei die Zeitspanne zwischen Beendigung der Zugabe der Aminoalkyltrialkoxysilane und Beginn des Abdestillierens des Alkylalkohols weniger als 5 Minuten nach Erreichen der bevorzugten Destillationsbedingungen, vorzugsweise weniger als 3 Minuten, besonders bevorzugt weniger als 1 Minute beträgt.

12. Verfahren nach einem der vorstehenden Ansprüche, wobei die wässrige Lösung einen Gesamtgehalt an freiem und gebundenen Alkylalkohol von kleiner gleich 0,7 Gew.-%, bezogen auf die Zusammensetzung, aufweist, gemessen nach Hydrolyse einer Probe der wässrigen Lösungen mit Schwefelsäure und nachfolgender Neutralisation mit wässriger Sodalösung mittels Gaschromatographie.

13. Verfahren nach einem der vorstehenden Ansprüche, wobei die wässrige Lösung einen Trübwert von 5 FNU oder weniger, vorzugsweise 3 FNU oder weniger, besonders bevorzugt von 2 FNU oder weniger, am meisten bevorzugt von 1 FNU oder weniger gemessen nach ISO 7027 aufweist.

## Claims

1. Process for preparing aqueous hydrolysates of aminoalkyltrialkoxysilanes by

   (a) initially charging water, optionally heating,
   (b) adding hydrolysable silanes consisting of at least one aminoalkyltrialkoxysilane in an amount which provides a molar ratio of water to total amount of aminoalkyltrialkoxysilane of 10.5 to 20:1, and
   (c) distilling off the alkyl alcohol formed in the reaction,

   wherein the solids content in an aqueous composition thus prepared is 30% to 55% by weight, based on the composition, and the aqueous solution has a total content of free and bound alkyl alcohol of not more than 1% by weight, based on the composition.

2. Process according to Claim 1, wherein the aminoalkyltrialkoxysilanes are selected from compounds of the general formula

$$HR^1N - R^2 - Si(OR^3)_3$$

   in which

   $R^1$ is selected from H or a $C_1$-$C_4$-alkyl group;
   $R^2$ is selected from a bivalent $C_1$-$C_4$-alkyl group; and
   $R^3$ is selected from a $C_1$-$C_3$-alkyl group.

3. Process according to Claim 2, wherein $R^1$ is H.

4. Process according to either of Claims 2 and 3, wherein $R^2$ is a bivalent propyl group $[-(CH_2)_3-]$ and $R^3$ is selected from methyl and ethyl.

5. Process according to any of Claims 1 to 4, wherein the aminoalkyltrialkoxysilane is selected from 3-aminopropyltriethoxysilane or 3-aminopropyltrimethoxysilane or mixtures thereof; the preferred aminoalkyltrialkoxysilane is especially 3-aminopropyltriethoxysilane.

6. Process according to any of the preceding claims, wherein the solids content in an aqueous composition thus prepared is 40% to 55% by weight, especially 50% to 53% by weight, based on the composition.

7. Process according to any of the preceding claims, wherein the molar ratio of water to the total amount of aminoalkyltrialkoxysilane is 11 to 18:1, here including the molar ratios of 12:1, 13:1, 14:1, 15:1, 16:1, 17:1.

8. Process according to any of the preceding claims, wherein the duration of the aminoalkyltrialkoxysilane addition in step (b) is 20 to 240 minutes, preferably 30 to 120 minutes, more preferably 35 to 90 minutes, most preferably 40 to 60 minutes.

9. Process according to any of the preceding claims, wherein the internal reactor temperature on commencement of the addition of the aminoalkyltrialkoxysilanes in step (b) is 25°C to 50°C, preferably 30°C to 45°C, and the internal reactor temperature at the end of the addition of the aminoalkyltrialkoxysilanes in step (b) is 50°C to 80°C, preferably 55°C to 70°C, more preferably 55°C to 65°C.

10. Process according to any of the preceding claims, wherein the distillative removal of the alkyl alcohol is commenced immediately after the addition of the aminoalkyltrialkoxysilanes has ended.

11. Process according to any of the preceding claims, wherein the period of time between the end of the addition of the aminoalkyltrialkoxysilanes and commencement of the distillative removal of the alkyl alcohol is less than 5 minutes after attainment of the preferred distillation conditions, preferably less than 3 minutes, more preferably less than 1 minute.

12. Process according to any of the preceding claims, wherein the aqueous solution has a total content of free and bound alkyl alcohol of not more than 0.7% by weight, based on the composition, measured after hydrolysis of a sample of aqueous solutions with sulphuric acid and subsequent neutralization with aqueous sodium carbonate solution by means of gas chromatography.

13. Process according to any of the preceding claims, wherein the aqueous solution has a turbidity value of 5 FNU or less, preferably 3 FNU or less, more preferably of 2 FNU or less, most preferably of 1 FNU or less, measured according to ISO 7027.

## Revendications

1. Procédé de fabrication d'hydrolysats aqueux d'aminoalkyltrialcoxysilanes par

   (a) chargement initial d'eau, éventuellement chauffage,
   (b) ajout de silanes hydrolysables constitués d'au moins un aminoalkyltrialcoxysilane en une quantité qui ajuste un rapport molaire entre l'eau et la quantité totale d'aminoalkyltrialcoxysilane de 10,5 à 20 sur 1, et
   (c) élimination par distillation de l'alcool alkylique formé lors de la réaction,

   la teneur en solides d'une composition aqueuse ainsi fabriquée étant de 30 à 55 % en poids, par rapport à la composition, la solution aqueuse présentant une teneur totale en alcool alkylique libre et lié inférieure ou égale à 1 % en poids, par rapport à la composition.

2. Procédé selon la revendication 1, dans lequel les aminoalkyltrialcoxysilanes sont choisis parmi les composés de formule générale

   $$HR^1N\text{-}R^2\text{-}Si(OR^3)_3,$$

   dans laquelle

   $R^1$ est choisi parmi H ou un groupe alkyle en $C_1$-$C_4$ ;
   $R^2$ est choisi parmi un groupe alkyle en $C_1$-$C_4$ bivalent ;
   et
   $R^3$ est choisi parmi un groupe alkyle en $C_1$-$C_3$.

3. Procédé selon la revendication 2, dans lequel $R^1$ est H.

4. Procédé selon l'une quelconque des revendications 2 ou 3, dans lequel $R^2$ est un groupe propyle bivalent [-$(CH_2)_3$-] et $R^3$ est choisi parmi méthyle et éthyle.

5. Procédé selon l'une quelconque des revendications 1 à 4, dans lequel l'aminoalkyltrialcoxysilane est choisi parmi le 3-aminopropyltriéthoxysilane ou le 3-aminopropyltriméthoxysilane ou leurs mélanges, le 3-aminopropyltriéthoxysilane étant notamment préféré en tant qu'aminoalkyltrialcoxysilane.

**6.** Procédé selon l'une quelconque des revendications précédentes, dans lequel la teneur en solides d'une composition aqueuse ainsi fabriquée est de 40 à 55 % en poids, notamment de 50 à 53 % en poids, par rapport à la composition.

**7.** Procédé selon l'une quelconque des revendications précédentes, dans lequel le rapport molaire entre l'eau et la quantité totale d'aminoalkyltrialcoxysilane est de 11 à 18 sur 1, y compris les rapports molaires 12:1, 13:1, 14:1, 15:1, 16:1, 17:1.

**8.** Procédé selon l'une quelconque des revendications précédentes, dans lequel la durée de l'ajout de l'aminoalkyltrialcoxysilane à l'étape (b) est de 20 à 240 minutes, de préférence de 30 à 120 minutes, de manière particulièrement préférée de 35 à 90 minutes, de manière préférée entre toutes de 40 à 60 minutes.

**9.** Procédé selon l'une quelconque des revendications précédentes, dans lequel la température intérieure du réacteur au début de l'ajout des aminoalkyltrialcoxysilanes à l'étape (b) est de 25 °C à 50 °C, de préférence de 30 °C à 45 °C, et la température intérieure du réacteur à la fin de l'ajout des aminoalkyltrialcoxysilanes à l'étape (b) est de 50 °C à 80 °C, de préférence de 55 °C à 70 °C, de manière particulièrement préférée de 55 °C à 65 °C.

**10.** Procédé selon l'une quelconque des revendications précédentes, dans lequel l'élimination par distillation de l'alcool alkylique est débutée immédiatement après la fin de l'ajout des aminoalkyltrialcoxysilanes.

**11.** Procédé selon l'une quelconque des revendications précédentes, dans lequel la durée entre la fin de l'ajout des aminoalkyltrialcoxysilanes et le début de l'élimination par distillation de l'alcool alkylique est de moins 5 minutes après avoir atteint les conditions de distillation préférées, de préférence de moins de 3 minutes, de manière particulièrement préférée de moins de 1 minute.

**12.** Procédé selon l'une quelconque des revendications précédentes, dans lequel la solution aqueuse présente une teneur totale en alcool alkylique libre et lié inférieure ou égale à 0,7 % en poids, par rapport à la composition, mesurée après l'hydrolyse d'un échantillon des solutions aqueuses avec de l'acide sulfurique, puis la neutralisation avec une solution de soude aqueuse par chromatographie gazeuse.

**13.** Procédé selon l'une quelconque des revendications précédentes, dans lequel la solution aqueuse présente une valeur de trouble de 5 FNU ou moins, de préférence de 3 FNU ou moins, de manière particulièrement préférée de 2 FNU ou moins, de manière préférée entre toutes de 1 FNU ou moins, mesurée selon ISO 7027.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 675128 A
- EP 716127 A
- EP 997469 A
- EP 1031593 A
- WO 200903538 A
- DE 10333941 A1
- EP 1017880 A
- US 3810843 A
- US 6512132 A
- US 6512132 B
- EP 1031593 A2

**In der Beschreibung aufgeführte Nicht-Patentliteratur**

- **ZENGPING ZHANG et al.** Journal of Applied Polymer Science. 2007, 2608-2614